# EUROPEAN PATENT APPLICATION

(11) **EP 1 371 986 A1**
(43) Date of publication of application: **17.12.2003**
(21) Application number: 02012583.7
(22) Date of filing: 06.06.2002
(51) Int. Cl.: G01N 33/68, C07K 16/18

(54) **Diagnosis of Alzheimer's disease based on the hAbeta42:hAbeta40 ratio**

(71) Applicant: ABETA GmbH, 69120 Heidelberg (DE)
(72) Inventor: Hartmann, Tobias, Dr., D-69120 Heidelberg (DE); Eikenberg, Oliver, Dr., D-69221 Dossenheim (DE); Antz, Christof, Dr., D-72070 Tübingen (DE)
(74) Representative: Schüssler, Andrea, Dr.

(57) **Abstract**

Described is a method for the diagnosis of Alzheimer's Disease (AD) or of a risk for developing said disease which is based on the determination of the ratio of hAß42:hAß40 (Aß42/40-ratio). A lower Aß42/40-ratio as compared to the normal ratio is indicative for AD or for a risk for developing AD. Moreover, kits are described suitable for carrying out said diagnostic method.

SEQUENCE LISTING

<110> ABETA GmbH

<120> Assay for the diagnosis of Alzheimer's disease based on
      the determination of the ratio of hAß42:hAß40

<130> A3025

<140> EP 02012583.7
<141> 2002-06-06

<160> 3

<170> PatentIn Ver. 2.1

<210> 1
<211> 8
<212> PRT
<213> Artificial Sequence

<220>
<223> Description of Artificial Sequence: Antigenic
      peptide

<400> 1
Met Val Gly Gly Val Val Ile Ala
  1               5

<210> 2
<211> 8
<212> PRT
<213> Artificial Sequence

<220>
<223> Description of Artificial Sequence: Antigenic
      peptide

<400> 2
Gly Leu Met Val Gly Gly Val Val
  1               5

<210> 3
<211> 9
<212> PRT
<213> Artificial Sequence

<220>
<223> Description of Artificial Sequence: Antigenic
      peptide

<400> 3
Cys Met Val Gly Gly Val Val Ile Ala
  1               5

## Description

The present invention relates to a method for the diagnosis of Alzheimer's Disease (AD) or of a risk for developing said disease which is based on the determination of the ratio of hAβ42:hAβ40 (Aβ2/40-ratio). A lower Aβ42/40-ratio as compared to the normal ratio is indicative for AD or for a risk for developing AD. The present invention also provides kits suitable for carrying out said diagnostic method.

For the diagnosis of Alzheimer's Disease (AD) and for distinguishing AD from other dementia various methods or combinations thereof are used. These diagnostic methods comprise the "scanning" of the brain using computer tomography (CT), magnetic resonance imaging (MRI) or positron emission tomography (PET) in order to visualize abnormalities in the brain. Another diagnostic approach is the mini-mental state examination (MMSE) which is based on the testing of particular cognitive problems (short-term memory, long-term memory, repetition tests), thus, allowing a distinct evaluation of the patient's capability as regards resolving of problems, linguistic competence etc. According to neurologists, geriatricians and psychiatrists who are familiar with a variety of dementia this test allows a precise diagnosis. Thus, MMSE forms the basis of current diagnosis.

An additional diagnostic method is based on the determination of compounds which are characteristic of the disease (biochemical markers, biomarkers) in body fluids like blood, liquor, urine etc. using selective laboratory tests. This approach allows the determination of changes of the concentrations due to the disease - even early in the course of the disease, when clinical symptoms might be mild and vague. However, this assay is problematic, since low concentrations of biochemical markers have to be determined selectively for early diagnosis and for exactly distinguishing between AD and other dementia. In order to enhance diagnostic accuracy, particular biochemical markers like the β-amyloid protein and the tau protein are used. The cerebrospinal fluid (CSF) level of tau has been suggested to reflect neuronal and axonal degeneration or possibly formation of neurofibrillary tangles. Accordingly, tau is regarded as a general biomarker for a neuronal dysfunction (Creutzfeldt-Jacob, AD etc.).

Currently, amyloid β (Aβ) is regarded as the most important diagnostic parameter, since this peptide is responsible for the plaque like extracellular depositions (amyloidosis) in the brain which is a characteristic feature early in the course of AD. β-amyloid is generated by the degradation of the cell membrane protein APP (amyloid precursor protein) by particular enzymes, i.e. α-, β- and γ-secretases. The biological function of cellular APP still remains enigmatic. APP is part of a superfamily from which sixteen homologous amyloid precursorlike proteins (APLP) and APP species homologues are derived. APP is an ubiquitous glycoprotein expressed highly in neurons. APP deficient mice are viable and develop normally, but they display minor defects that differ according to the null strain, e.g., copper levels are increased in the cerebral cortex and liver of APP and APLP2 knockout mice. Thus, APP is not required for the expression of a critical cell function, but may be involved in the modulation of neuronal functions at the cellular level. Cleavage of APP by β-secretases results in the formation of soluble fragments of APP, which are further degraded to various amyloid species by γ-secretases. So far, various amyloid species could be identified according to their amino acid sequences and classified according to different lengths (39 to 42 amino acids)and N-termini (e.g., Val 40 = Aβ x-40; Ala 42 = Aβ x-42, Val 43 = Aβ x-43). These peptides are also present in healthy individuals, however, in case of AD etc. their concentrations are different which might be diagnostically relevant. Unfortunately, even in healthy individuals, the concentrations of these biomarkers vary due to age, sex, nutrition, diabetes mellitus, function of the liver or kidneys, oxidative stress as well as genetic factors (e.g. ApoE-gene mutations), thus, impeding exact diagnosis. So far, none of these biomarkers has been accepted for clinical routine diagnostic.

Since some years the parameter amyloid beta (Aβ), in particular Aβ40, is used as a specific biomarker for the diagnosis of AD. The determination of the presence or concentration of biomarkers like Aβ is mainly based on biochemical assays, e.g. immunochemical assays. In liquor, Aβ40 (which is involved in regulation) is the predominant species (about 90%) compared to Aβ42 (about 5-10%). In contrast to other Aβ species, Aβ42 is capable of forming aggregates.

In a recent study, the levels of Aβ40 and Aβ42 of AD patients and of healthy individuals were compared. It could be shown that there were no differences in both groups as regards the levels of Aβ40, however, AD patients showed decreased levels of Aβ42. Unfortunately, the absolute concentrations of Aβ42 observed in various AD patients differ dramatically, i.e. in different studies the following mean values were found: 280 pg/ml, 384 pg/ml, 497 pg/ml, 537 pg/ml, 740 pg/ml and 835 pg/ml. These variations of values of absolute concentrations show that the data obtained have been affected by various factors (state of the sample, assay system used, etc.). Accordingly, the establishing of limit values which is required for the development of a reliable diagnostic assay is almost impossible. Moreover, in the course of sporadic AD disease various levels of amyloid could be observed. In a recent study, it was found that during an early stage of the disease the concentration of Aβ40 decreased. By contrast, the concentration of Aβ42 increased, however, with progression of the disease the Aβ42 levels decreased resembling normal values. Moreover, it was found for a healthy control group that the levels of Aβ42 decreased depending on age, however, no significant difference could be observed between AD patients and patients having other dementia. Accordingly, the determination of the concentration of Aβ42 only is not sufficient for precise diagnosis of AD. In other words, at present, there is no reliable marker available for diagnosing AD or for diagnosing a elevated risk for developing AD.

Thus, the technical problem underlying the present invention is to provide a marker for the reliable (early) diagnosis of Alzheimer's disease (AD) or of a predisposition for said disease.

The solution to said technical problem is achieved by providing the embodiments characterized in the claims.

It has been, surprisingly, found that a reliable diagnosis of AD (or the progression of the disease or the monitoring of the effect of therapeutic drugs) which is independent from the state of the sample, age of the patient, sex etc. can be achieved by determining the ratio of amyloid Aβ42 concentration to amyloid Aβ40 concentration (Aβ42/40-ratio).

### BRIEF DESCRIPTION OF THE DRAWINGS

### Figure 1: Diagram showing the results of an ELISA using the anti-Aβ42-antibody G2-11 versus the anti-Aβ42-antibody G2-13

### Figure 2: Comparison of the diagnostic parameters MMSE, Aβ42, Aβ40 and Aβ42/40-ratio

In the table, the values for MMSE, Aβ42, Aβ40 and Aβ42/40-ratio are given in correlation to medical diagnosis (listed according to declining values).

### Figure 3: Diagram representing the results shown in Figure 2

The present invention, thus, relates to a method for the diagnosis of Alzheimer's Disease (AD) or of a risk for developing said disease, comprising the following steps:
(a)contacting a sample with probes specifically binding to hAβ42 and hAβ40, respectively; and
(b) determining the amounts of hAβ42 and hAβ40 in said sample, wherein a lower ratio of hAβ42 : hAβ40 (Aβ42/40-ratio) as compared to the normal ratio is indicative for AD or for a risk for developing AD.

The term "normal ratio" as used herein corresponds to a Aβ42/40-ratio which can be found in healthy individuals and which is determined by using the average values obtained from at least 10, preferably from at least 100 individuals.

The determination of the hAβ42/40-ratio is useful for prognosis, early diagnosis, for monitoring the progression of the disease and for the diagnosis of a late state of the disease.

Suitable samples for carrying out the diagnostic method of the invention are cerebrospinal fluid (CSF), blood, serum, plasma or urine. The sample is taken from the patient using routine methods, e.g. CSF is taken by lumbar puncture. The preferred sample is CSF.

In a preferred embodiment of the diagnostic method the ratio of hAβ42:hAβ40 (Aβ42/40 ratio) is less or equal 0.8 times compared to the mean value of the normal ratios obtained by a population of at least 10 healthy individuals. More preferably, the Aβ42/40 ratio is less or equal 0.6 times compared to the mean value of the normal ratios obtained by a population of at least 10 healthy individuals.

Any method allowing the determination of the levels of hAβ42 and hAβ40, respectively, in a sample can be used in the method of the present invention, e.g. immunological assays, size separation, e.g. by gel filtration, amino acid analyses etc. with immunological assays being preferred.

In an even more preferred embodiment of the method of the present invention, the determination of the Aβ42/40-ratio is carried out by an immunological assay using antibodies specific for hAβ42 and hAβ40, respectively, i.e., the anti-hAβ42-antibody does not substantially cross-react with hAβ40 and, vice versa, the anti-hAβ40-antibody does not substantially cross-react with hAβ42. Suitable antibodies are commercially available, e.g. the antibody G2-11 (specific for Aβ42) and G2-10 (specific for Aβ40), both described in Ida et al., J. Biol. Chem. 271 (1996), 22908-22914 and commercially available from ABETA GmbH, Heidelberg, Germany. A particular preferred antibody against Aβ42 is the antibody G2-13 which is described in Example 1 below and commercially available from ABETA GmbH, Heidelberg, Germany.

The term "antibody" as used herein, preferably, relates to antibodies which consist essentially of pooled monoclonal antibodies with different epitopic specificities, as well as distinct monoclonal antibody preparations. Monoclonal antibodies are made from an antigen containing fragments of the hAβ42 peptide and hAβ40, respectively, by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody. Moreover, these include chimeric, single chain, and humanized antibodies.

In a particularly preferred embodiment of the method of the present invention the anti-hAβ42-antibody is directed to the epitope of hAβ42 at amino acid positions 35-42 (MVGGWIA) and the anti-hAβ40-antibody is directed to the epitope of hAβ40 at amino acid positions 33-40 (GLMVGGVV). These antibodies can be prepared, e.g., according to the procedure described in Ida et al., 1996.

The term "epitope" as used herein is meant to include any determinant responsible for specific interaction with an antibody molecule. Epitopic determinants usually consist of chemically active surface groupings of molecules such as amino acids or sugar side chains and have specific three-dimensional characteristics as well as specific charge characteristics.

The probes, e.g. an antibody, can be detectably labelled, for example, with a radioisotope, a bioluminescent compound, a chemiluminescent compound, a fluorescent compound, a metal chelate, or an enzyme (e.g. horse-radish peroxidase, alkaline phosphatase, β-galactosidase, malate dehydrogenase, glucose oxidase, urease, catalase etc.) which, in turn, when later exposed to a substrate will react to the substrate in such a manner as to produce a chemical moiety which can be detected. The probes can also be immobilized on an insoluble carrier, e.g. glass, polystyrene, polypropylene, polyethylene, dextran, nylon, natural and modified celluloses, polyacrylamides, agarose and magnetic beads.

Examples of immunoassays suitable for the method of the present invention are competitive or sandwich assays, such as the enzyme linked immunosorbent assay (ELISA), the radioimmunoassay (RIA) or Western Blots. Suitable antibody assay labels are known in the art and include enzyme labels, such as, glucose oxidase, and radioisotopes, such as iodine (¹²⁵I, ¹²¹I), carbon (¹⁴C), sulfur (³⁵S), tritium (³H), indium (¹¹²In), and technetium (⁹⁹mTc), and fluorescent labels, such as fluorescein and rhodamine.

In a further preferred embodiment, the method of the present invention is carried out in an ELISA format with a sandwich-ELISA format being even more preferred.

Suitable ELISA and sandwich-ELISA formats are well known to the person skilled in the art and, furthermore, described in the examples below.

The method of the present invention can be modified, e.g., by additionally determining the concentration of further biomarkers (ApoE, htau, Aβ38) which might, in certain cases, further improve the diagnostic value of the method of the invention.

For use in the diagnostic research discussed above, kits are also provided by the present invention. Such kits are useful for the determining the amounts of hAβ42 and hAβ40, respectively, in a sample, and comprise specific probes for detection of hAβ42 and hAβ40. The probes can be detectably labeled as described above. In a preferred embodiment, said kit contains a first monoclonal anti-hAβ42-antibody directed to the epitope of hAβ42 at amino acid positions 35-42 (MVGGVVIA), preferably the antibody G2-13, and a second monoclonal anti-hAβ40-antibody directed to the epitope of hAβ40 at amino acid positions 33-40 (GLMVGGVV) and, preferably allows diagnosis, e.g., by ELISA and contains the antibodies bound to a solid support, for example, a polystyrene microtiter dish or nitrocellulose paper, using techniques known in the art. Alternatively, said kits are based on a RIA and contain said antibodies marked with a radioactive isotope. In a preferred embodiment of the kit of the invention the antibodies are labeled with enzymes, fluorescent compounds, luminescent compounds, ferromagnetic probes or radioactive compounds.

The following Examples illustrate the invention.

### Example 1: Generation of the anti-hAβ42-antibody G2-13

The antibody was generated essentially according to the method described in Ida et al., 1996. The peptide used as antigen had the following amino acid sequence: CMVGGVVIA. The isotype of G2-13 is IgG1 and it specifically recognizes βA₄1-42, C-terminal βA₄C42. It does not show cross-reactivity with the peptides Aβ38, Aβ39 and Aβ40. Cross-reactivity with Aβ43 and Aβ44 is about 10%.

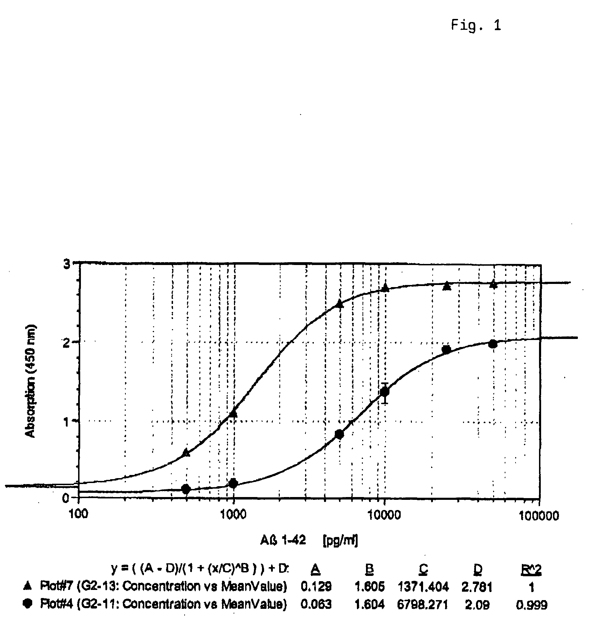

The characteristics of the antibody G2-13 as regards sensitivity were compared with G2-11. Figure 1 shows the results of the determination of amyloid β42 concentrations using G2-13 and G2-11, respectively. The C50-value characterizing sensitivity is given. A shift of the curve to the left indicates an increase of sensitivity. It can be inferred from Figure 1 that the sensitivity of the antibody G2-13 is 2-3 times higher compared with antibody G2-11.

### Example 2: Development of a hAβ42/40-specific sandwich ELISA

ELISA (enzyme-linked immunosorbent assay) is a well known method for the routine diagnosis of particular molecules which is based on a solid phase immunoassay. The sandwich ELISA described below allows the selective and sensitive detection of hAβ42 and hAβ40, respectively, and, thus, the determination of the hAβ42/40-ratio.

Briefly, ninety-six-wells plates (Maxisorp™, Nunc, Denmark) were coated with the capture-antibody W0-2 described in Ida et al., (1996) (commercially available from ABETA GmbH, Heidelberg, Germany), 5 µg/ml, in coating buffer (50 mM Carbonate Buffer, pH 9.5 - 9.7) overnight at 4°C. The following day, plates were blocked with ELISA blocking agent (Stabil Coat, Surmodics, Minnesota, USA) for 2 hours at room temperature on a plate shaker. Standard curves were prepared from a stock solution (1 µg/ml) of synthetic amyloid β1-40 peptide and stored at -70°C. The standard curves were diluted in ELISA buffer (80 mM phosphate-buffered saline with 1% bovine serum albumin, 0,05% Tween 20™) to the concentrations in the test range from 10 to 2000 pg/ml. 50 µl secondary biotinylated (detection) antibody G2-13 and G2-11, for the hAβ42 assay, and G2-10, for the hAβ40 assay, together with 50 µl of CSF samples (and standards) were incubated on the plates overnight at 2-8°C. The day after, the plates were washed for 5 times with phosphate buffer (8 mM PBS, pH 7.4) and then 100 µl of horseradish peroxidase-conjugated streptavidin was allowed to bind for 30 min at room temperature on a plate shaker. Plates were developed by adding 100 µl of substrate solution (3,3',5,5'-tetramethylbenzidin; 1-Step-Ultra-TMB, PIERCE, Bonn, Germany). After 10-30 min, the substrate reaction was stopped by addition of 50 µl of 1 M H₂SO₄ per well, and the plates were read at 450 nm (reference wave length: 650 nm) on a plate reader (Emax, Molecular devices, Gothenburg, Sweden). The same standard curve batches were used on all plates, thus, allowing a quantitative determination of the levels of hAβ42 and hAβ40, respectively. Using this ELISA, concentrations in the range of 100 to 2000 pg/ml can be quantitatively determined. This range of concentrations comprises the hAβ concentrations in human liquor and, accordingly, the described ELISA is diagnostically highly useful. The detection limits for both assays were estimated to be in the range of about 100 pg/ml.

### Example 3: Comparison of the diagnostic parameters MMSE, Aβ42, Aβ40 and Aβ42/40-ratio

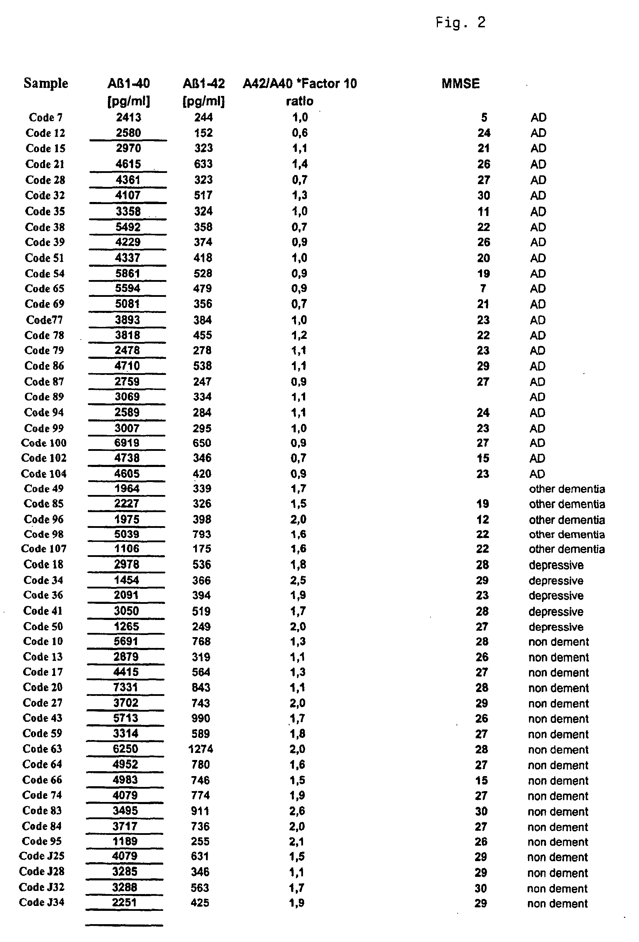

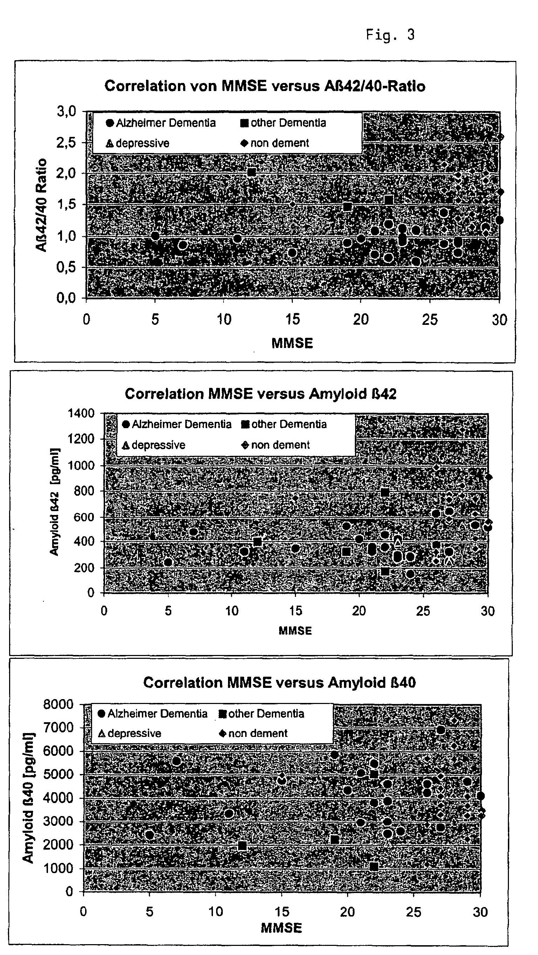

In a study comprising 52 patients the diagnostic parameter mini-mental state examination (MMSE) was compared with the biomarkers Aβ42, Aβ40 and Aβ42/40-ratio. For the determination of the concentrations of Aβ42 and Aβ40, the ELISA described in Example 2 was used. Figure 2 shows a comparison of the results obtained by the determination of the concentration of Aβ42 and Aβ40 and the calculation of the Aβ42/40-ratio with clinical diagnosis (MMSE). The results represented in Figure 2 are additionally shown in Figure 3 as a diagram (correlation of the data as regards Aβ42, Aβ40 and AB42/40-ratio with the MMSE-values after classification in particular groups (AD, other dementia, no dementia, depressive)).

The data obtained and presented in Figures 2 and 3 clearly show that when correlating the MMSE-values with Aβ40- and Aβ42-concentrations, respectively, a significant discrimination between the particular groups is not possible. However, when correlating the Aβ42/40-ratio with the MMSE-value a limit value (cut-off) of < 1.0 is obtained. A Aβ42/40-ratio of < 1.0 is in perfect agreement (100%) with clinical diagnosis (AD) and never gave a false positive diagnosis. The cut-off of 1.0 used in this study corresponds to a Aβ42/40 ratio, which is less or equal 0.8 times compared to the mean value of the normal ratios obtained by a population of at least 10 healthy individuals.

## Claims

1. A method for the diagnosis of Alzheimer's Disease (AD) or of a risk for developing said disease, comprising the following steps:
(a)contacting a sample with probes specifically binding to hAβ42 and hAβ40, respectively; and
(b) determining the amounts of hAβ42 and hAβ40 in said sample, wherein a lower ratio of hAβ42:hAβ40 (Aβ42/40-ratio) as compared to the normal ratio is indicative for AD or for a risk for developing AD

2. The method of claim 1, wherein the ratio of hAβ42:hAβ40 (Aβ42/40-ratio) is less or equal 0.8 times compared to the mean value of the normal ratios obtained by a population of at least 10 healthy individuals.

3. The method of claim 2, wherein the ratio of hAβ42:hAβ40 (Aβ42/40-ratio) is less or equal 0.6 times compared to the mean value of the normal ratios obtained by a population of at least 10 healthy individuals.

4. The method of any one of claims 1 to 3, wherein the sample is CSF.

5. The method of any one of claims 1 to 4, wherein the probes are antibodies specific for hAβ42 and hAβ40, respectively.

6. The method of claim 5, wherein the anti-hAβ1-42-antibody is directed to the epitope of hAβ42 at amino acid positions 35-42 (MVGGVVIA) and the anti-hAβ40-antibody is directed to the epitope of hAβ40 at amino acid positions 33-40 (GLMVGGVV).

7. The method of claim 5 or 6, wherein the antibodies are monoclonal antibodies.

8. The method of claim 5, wherein the anti-hAβ1-42-antibody is G2-13.

9. The method of any one of claims 5 to 8, wherein the method is carried out as ELISA.

10. The method of claim 9, wherein the ELISA is a sandwich-ELISA.

11. The method of any one of claims 1 to 10, comprising the determination of the concentration of at least one additional biomarker.

12. A kit suitable for carrying out the method according to any one of claims 1 to 11 comprising a first monoclonal anti-hAβ42-antibody directed to the epitope of hAβ42 at amino acid positions 35-42 (MVGGVVIA) and a second monoclonal anti-hAβ40-antibody directed to the epitope of hAβ40 at amino acid positions 33-40 (GLMVGGVV).
